# EUROPEAN PATENT APPLICATION

(11) **EP 0 974 321 A1**
(43) Date of publication of application: **26.01.2000**
(21) Application number: 99113695.3
(22) Date of filing: 16.07.1999
(51) Int. Cl.: A61F 9/007

(54) **Surgical instrument for universal use and particularly for orthopaedic use**

(30) Priority: 20.07.1998 IT MO980164
(71) Applicant: Sirius Medical S.r.l., 00195 Roma (IT)
(72) Inventor: Polidori, Claudio, 00196 Roma (IT)
(74) Representative: Modiano, Guido, Dr.-Ing.

(57) **Abstract**

A surgical instrument for universal use and particularly for orthopaedic use comprises a cylindrical hollow outer body having, at one end, a coaxially protruding stem-like extension for the guided internal passage of a reciprocating blade and having, at the opposite end, a grip provided with couplings for conventional means for dispensing actuation fluids and blade wetting fluids, the grip internally forming a seat for a fixed shutter which is crossed by corresponding ducts parallel to the longitudinal axis for the passage of at least, respectively, a fluid for actuating the blade, a liquid for wetting the active end of the blade and for aspirating the used liquid. The shutter forms, in the grip, a chamber for the reciprocating sliding of a pusher element which is connected to the base of the blade and can be actuated by means of the fluid in contrast with elastic means and has, at the opposite end, a recessed seat for dynamically accommodating a reservoir for the wetting liquid which can perform a reciprocating motion rigidly with the blade.

## Description

The present invention relates to a surgical instrument for universal use and particularly for orthopaedic use.

In the field of surgery it is necessary to have tools which are very small and extremely handy to use.

Because of their characteristics, these instruments are used when the space available for surgery is extremely confined and the surgical procedure requires very delicate work in order to avoid damage to patients.

An example is given by eye surgery, in which every movement must be calibrated with maximum precision.

Another field in which this care must be particularly accurate is orthopaedics, particularly in surgical procedures meant to treat disorders arising from vertebral damage or from slipped disks.

In the latter disorder it is often necessary to cut the portions of the nucleus polposus which, if compressed between the intervertebral disks, indeed cause said hernias, with all the associated discomfort for patients.

Conventional instruments are constituted by so-called handpieces, which are in practice a sort of cylinder whose dimensions are substantially similar to those of a fountain pen. The handpieces are provided with a point which protrudes from one of their ends and which is in turn provided with a blade which can perform an axial reciprocating motion, protrudes from an opening formed in the point and cuts whatever is placed inside the opening.

The reciprocating motion of the blade can occur through electric means and through pneumatic means, by means of corresponding externally powered devices included in the handpiece body.

The supply of physiological solution and/or disinfectant which, by means of a supply duct and a drainage duct, must constantly wet the cutting region also arrives from an external source.

The wetting is an indispensable prerequisite for using the handpiece; however, the necessary supply and return hoses, which are generally integrated but arranged laterally with respect to the body of the handpiece, significantly increase its overall dimensions and therefore limit its ergonomics and practicality in use.

Moreover, in the regions between the reciprocating parts and the fixed parts, in the case of pneumatic actuation, overall tightness has been found to be highly unstable in conventional handpieces, sometimes causing accidental and undesirable mixing of the actuation fluid and the physiological solution.

The aim of the present invention is to solve the above-cited problems of the prior art, by providing a surgical instrument for universal use and particularly for orthopaedic use which is ergonomic, extremely compact, has no problems between the fixed parts and the moving parts that constitute it, and is easy to use and maintain.

This aim and other objects are all achieved by a surgical instrument for universal use and particularly for orthopaedic use comprising a cylindrical hollow outer body and having, at one end, a coaxially protruding stem-like extension for the guided internal passage of a reciprocating blade and having, at the opposite end, a grip provided with couplings for conventional means for dispensing actuation fluids and blade wetting fluids, characterized in that the grip internally forms a seat for a fixed shutter which is crossed by corresponding ducts parallel to the longitudinal axis for the passage of at least, respectively, a fluid for actuating the blade, a liquid for wetting the active end of the blade and for aspirating the used liquid, the shutter forming, in the grip, a chamber for the reciprocating sliding of a pusher element which is connected to the base of the blade and can be actuated by means of the fluid in contrast with elastic means and has, at the opposite end, a recessed seat for dynamically accommodating a reservoir for the wetting liquid which can perform a reciprocating motion rigidly with the blade.

Further characteristics and advantages will become apparent from the description of a preferred embodiment of a surgical instrument for universal use and particularly for orthopaedic use, illustrated only by means of non-limitative example in the accompanying drawings, wherein:

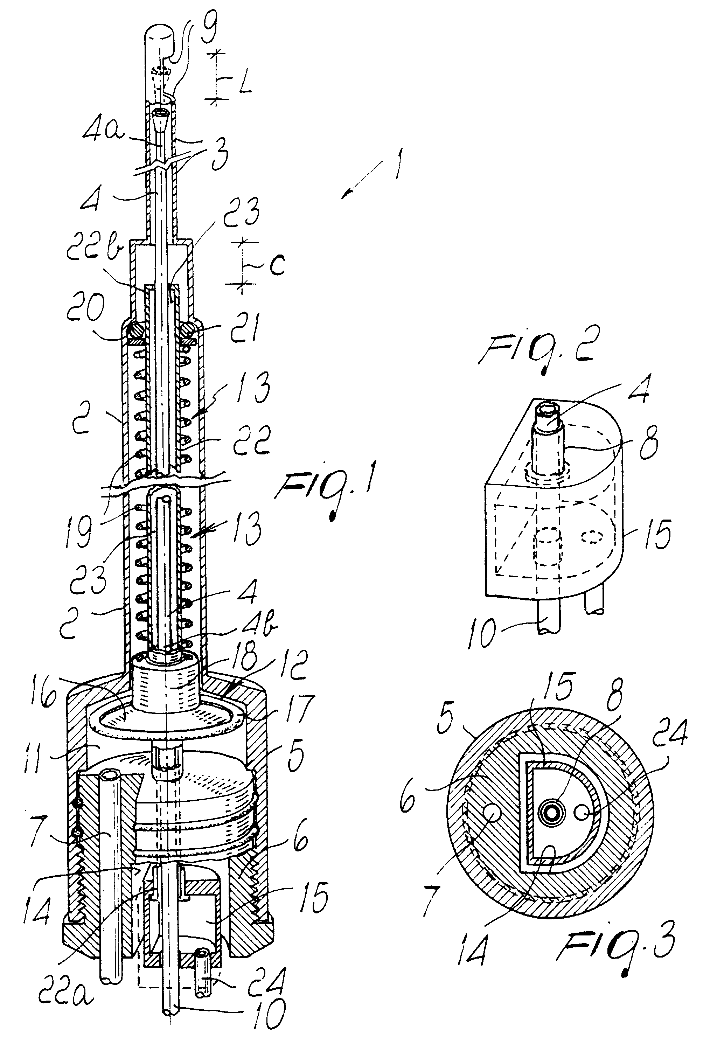

Figure 1 is a longitudinal sectional view of the invention;
Figure 2 is a detail view of a reservoir for containing wetting liquid;
Figure 3 is a bottom view of the invention.

With reference to the above figures, the reference numeral 1 generally designates a surgical instrument for universal use and particularly for orthopaedic use, comprising an internally hollow cylindrical outer body 2 having, at one end, a stem-like extension 3 which protrudes coaxially.

A reciprocating blade 4 slides and is guided inside the body 2 and the associated extension 3.

The body 2 has, at the opposite end with respect to the one from which the extension 3 protrudes, a grip 5 provided with a set of couplings, one for conventional means for dispensing a fluid which moves the blade 4 and a second one for supplying a liquid for wetting the blade 4.

The grip 5 internally forms a hollow seat for accommodating a fixed shutter 6 crossed by corresponding ducts 7 and 8 which are parallel to the longitudinal axis and through which the fluid for moving the blade 4 and the liquid for wetting the active end 4a of the blade flow.

Advantageously, the blade 4 is cylindrical and internally hollow and its end 4a is adapted to cross, during its movement, an opening 9 formed proximate to the tip of the extension 3.

Used liquid is aspirated in a conventional way through the blade 4 and is conveyed to a discharge by means of a further duct 10 being concentric to the wetting duct 8.

The shutter 6 forms, inside the grip 5, a chamber 11 in which a pusher element 12 can slide with a reciprocating motion; the pusher element is connected to the base of the blade 4 and can be actuated by means of the fluid in contrast with elastic means 13.

The shutter 6 further has, on the opposite side with respect to the blade 4, a recessed seat 14 for dynamically accommodating a reservoir 15 for the wetting liquid. The reservoir is rigidly coupled to the base of the blade 4.

The sliding chamber 11 is arranged at the region where the grip 5 is attached to the cylindrical body 2 and the pusher 12 is constituted by a circular diaphragm 16 which is perimetrically provided with means for providing a seal with the sliding chamber 11, the means are constituted in practice by an annular gasket 17 made of elastomeric material which is centrally provided with a protruding neck 18 to which the corresponding base 4b of the blade 4 is coupled.

These elastic contrast means 13 are constituted by at least one helical spring 19 which is coaxially accommodated inside the cylindrical body 2 and is wrapped around the blade 4. The ends of the spring 19 are contained in abutment between the attachment shoulder 20 of the stem-like extension 3, with a sealing ring 21 interposed, and the circular diaphragm 16.

In the preferred embodiment of the surgical instrument 1, the blade 4 is perimetrically surrounded by a blade guiding cannula 22, in which the base 22a is coaxially rigidly coupled to the neck 18 of the circular diaphragm 16 and the top end 22b is contained in the cylindrical body 2. The blade guiding cannula 22 can move rigidly with the blade 4 over a stroke C which is substantially equal to the length L of the opening 9.

A cylindrical annular passage 23 is intentionally left between the blade 4 and the blade guiding cannula 22 in order to allow the flow of the wetting liquid directed toward the active end 4a of the blade.

The blade guiding cannula 22 further passes fully through the fixed shutter 6 and protrudes into the reservoir 15 for the wetting liquid.

Another hose 24 from outside is further connected to the tank 15 and the wetting liquid is supplied to the reservoir 15 through the hose.

The operation of the invention is as follows: a pulsating fluid arrives in the chamber 11 through the duct 7, acting on the circular diaphragm 16, which by moving up and down in contrast with the spring 19 provides the reciprocating motion to the blade 4 and to the blade guiding cannula 22.

The gasket 17 prevents the fluid (in practice, compressed air) from flowing out of the chamber 11.

At the same time, the wetting liquid (usually constituted by physiological solution) that arrives from the reservoir 15 passes between the blade and the blade guiding cannula 22. By being rigidly coupled to the base end 22a of the cannula 22, the reservoir, in addition to storing a reserve of the liquid, follows the reciprocating motion of the blade 4, moving inside the recessed seat 14 formed in the fixed shutter 6 that closes the grip 5.

When the wetting liquid has reached the active end 4a of the blade 4, where it performs its action, it is aspirated through the blade to be sent to the discharge by means of an additional duct 10 which is conceptually the extension of the cannula 22 beyond the reservoir 15.

In practice, therefore, the wetting liquid, by flowing along a forced path, can never make contact, even accidentally, with the fluid for moving the diaphragm 16 and therefore the blade 4.

Moreover, the concentric arrangement of the ducts allows to provide a body for the surgical instrument 1 which is extremely slender and ergonomic and therefore easy to handle.

It has thus been observed that the described invention achieves the intended aim.

The invention thus conceived is susceptible of numerous modifications and variations, all of which are within the scope of the inventive concept.

All the details may further be replaced with other technically equivalent elements.

In the practical embodiment of the invention, the materials used, as well as the shapes and the dimensions, may be any according to requirements without thereby abandoning the scope of the protection of the appended claims.

The disclosures in Italian Patent Application No. MO98A000164 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A surgical instrument for universal use and particularly for orthopaedic use, comprising a cylindrical hollow outer body having, at one end, a coaxially protruding stem-like extension for the guided internal passage of a reciprocating blade and having, at the opposite end, a grip provided with couplings for conventional means for dispensing actuation fluids and blade wetting fluids, characterized in that said grip internally forms a seat for a fixed shutter which is crossed by corresponding ducts parallel to the longitudinal axis for the passage of at least, respectively, a fluid for actuating the blade, a liquid for wetting the active end of the blade and for aspirating the used liquid, the shutter forming, in the grip, a chamber for the reciprocating sliding of a pusher element which is connected to the base of the blade and can be actuated by means of the fluid in contrast with elastic means and has, at the opposite end, a recessed seat for dynamically accommodating a reservoir for the wetting liquid which can perform a reciprocating motion rigidly with the blade.

2. The surgical instrument according to claim 1, characterized in that the sliding chamber is arranged at the point where the grip is attached to the cylindrical body.

3. The surgical instrument according to claim 1, characterized in that the pusher element is constituted by a circular diaphragm which is perimetrically provided with means for forming a seal with the sliding chamber and is centrally provided with a protruding neck for the coupling of the corresponding base of the blade.

4. The surgical instrument according to claim 3, characterized in that the sealing means are constituted by an annular gasket made of elastomeric material.

5. The surgical instrument according to claims 1 and 3, characterized in that the elastic contrast means are constituted by at least one helical spring which is coaxially accommodated inside the cylindrical body and is wound around the blade, the ends of the spring being contained in abutment between the attachment shoulder of the stem-like extension and the circular diaphragm.

6. The surgical instrument according to the preceding claims, characterized in that the blade is perimetrically surrounded by a blade guiding cannula, in which the base is coaxially rigidly coupled to the neck of the circular diaphragm and the upper end is contained in the cylindrical body, the blade guiding cannula being movable rigidly with the blade.

7. The surgical instrument according to the preceding claims, characterized in that a cylindrical annular passage for the flow of the wetting liquid is formed between the blade and the blade guiding cannula.

8. The surgical instrument according to claims 1, 6 and 7, characterized in that the blade guiding cannula passes through the fixed shutter and protrudes into the wetting liquid reservoir.
